# EUROPEAN PATENT APPLICATION

(11) **EP 2 647 373 A1**
(43) Date of publication of application: **09.10.2013**
(21) Application number: 13175166.1
(22) Date of filing: 11.05.2009
(51) Int. Cl.: A61K 31/13, A61K 31/4545, A61K 31/439, A61K 31/506, A61P 27/02

(54) **Methods for preventing the development of retinopathy by the oral administration of exo-S-mecamylamine.**

(30) Priority: 12.05.2008 US 52480 P
(62) Divisional of application: 09747285.6
(71) Applicant: Targacept, Inc., 100 North Main Street Suite 1510 Winston-Salem, NC 27101 (US); Toler, Steve, Winston-Salem, NC 27106 (US); Lippiello, Patrick M., Lewisville, NC 27023 (US); Cullison, Scott N., Winston-Salem, NC 27103 (US); Bencherif, Merouane, Winston-Salem, North Carolina 27106 (US)
(72) Inventor: Toler,, Steve, Winston-Salem, NC North Carolina 27106 (US); Lippiello,, Patrick M, Lewisville, NC North Carolina 27023 (US); Cullison,, Scott N, Winston-Salem, NC North Carolina 27103 (US); Bencherif,, Merouane, Winston-Salem, NC North Carolina 27106 (US)
(74) Representative: Potter Clarkson LLP

(57) **Abstract**

The invention provides exo-S-mecamylamine for use in oral administration to delay the onset or progression of retinopathy in a patient having an increased risk of developing retinopathy.

## Description

### BACKGROUND OF THE INVENTION

Although some macular dystrophies affecting younger individuals are sometimes referred to as macular degeneration, the term generally refers to age-related macular degeneration (AMD or ARMD).

The inner layer of the eye is the retina, which contains nerves that communicate sight, and behind the retina is the choroid, also called the choriocapillaries, which supplies blood to the retina. The retina consists of several layers, including (proceeding from exterior to interior) the Bruch's membrane, the retinal pigment epithelia, and the photoreceptor cell (rods and cones) layer.

The precursor to the development of AMD is called drusen. Drusen, thought to be a mixture of protein and lipid, occur in two forms, hard and soft. The presence of hard drusen, which are small and round in shape, yellow in color, and have well-defined boundaries, does not usually affect vision, is common in people at all ages, and is not considered a sign of age-related maculopathy. In the development of AMD, the drusen first evolve from hard to soft, increasing in number, size and confluence. The resulting soft drusen are pale yellow in color and large with ill defined margins. The presence of soft drusen may or may not adversely affect vision, but as the drusen accumulate in the central area of the retina (the macula), the likelihood of vision loss increases. People over the age of 55 with soft drusen larger than 63 micrometers (approximately half the width of a major retinal vein at the optic nerve head) are typically said to have age-related maculopathy and run a much increased risk of developing AMD. The risk is considerably higher when the drusen are large and numerous and associated with disturbance in the retinal pigmented epithelial cell layer under the macula.

The dry (nonexudative) form of AMD, also called central geographic atrophy, results from atrophy to the retinal pigment epithelial layer below the neuroretina, which causes vision loss through loss of photoreceptors (rods and cones) in the central part of the eye. The accumulation of drusen between the retina and the choroid can also result in the retina becoming detached. The wet (exudative) form of AMD, also called neovascular AMD, is the more severe form. In wet AMD, abnormal growth in the blood vessels of the choriod through the overlying Bruch's membrane and into and sometimes through the retinal pigment epithelial layer, ultimately leads to blood and protein leakage either immediately below or within the photoreceptors of the macula. The bleeding, leaking, and scarring from these blood vessels eventually causes irreversible damage to the photoreceptors and resulting vision loss. The blood vessels growth can also cause the retina to become detached. It can be treated with laser coagulation, and with medication that stops and sometimes reverses the growth of blood vessels.

Treatment for dry AMD typically includes vitamin supplements containing high doses of antioxidants, lutein and zeaxanthin, an attempt to slow the progression of dry AMD to wet AMD. The treatment of wet AMD typically involves administration of inhibitors of neovascularization in an attempt to slow the abnormal growth of the choriod blood vessels and their invasion of the overlying retinal layers.

Diabetic retinopathy is the result of microvascular changes in the retina. Small blood vessels, such as those in the eye, are especially sensitive to poor blood sugar control. Hyperglycemia damages the tiny blood vessels in the retina and choriod and makes them more permeable. During the initial stage, called non-proliferative or pre-proliferative DR, most people do not notice any change in their vision. It is often characterized by superficial retinal hemorrhages and cotton wool spots. A condition called macular edema can also occur. This is the result of fluid leaking from the blood vessels into the macula. The fluid makes the macula swell, which blurs vision.

As the disease increases in severity, non-proliferative DR becomes proliferative DR, in which the lack of oxygen in the retina causes fragile, new, blood vessels to grow along the retina and in the vitreous humour that fills the inside of the eye. These blood vessels can bleed, cloud vision, and destroy the retina. Vascular proliferation can also cause retinal detachment and neovascular glaucoma.

In addition to management of the underlying blood sugar instability, treatment of DR typically involves laser surgery, injection of corticosteroids and vitrectomy.

Retinopathies affect millions of people worldwide each year. In North America and Western Europe, AMD and DR are the leading causes of blindness. Currently, there are no effective agents to delay the onset of or reduce the development of these retinal diseases. Present modalities of treatment are implemented too late in the course of the disease, often require high risk expensive specialized procedures, and merely attempt to prevent further loss of vision. Although subjects at risk for developing AMD or DR are readily identifiable prior to the development of overt disease and vision loss, such subjects receive little or no treatment since there are few treatment options (antioxidant vitamins, better diabetes control). Soft drusen (yellow/white retinal deposits) are an early precursor to AMD. Individuals with significant soft drusen, and/or a family history of AMD should receive treatment to delay the onset or reduce the development of both dry and wet AMD. After 20 years of diabetes, nearly all patients with type 1 diabetes and >60% of patients with type 2 diabetes have some degree of retinopathy. Subjects with a history of diabetes, especially poorly controlled diabetes, should also receive therapy to delay the onset or reduce the development of retinopathy symptoms.

Currently available treatments for AMD and DR focus on the vascular complications associated with advanced disease. Such therapies do not delay the onset of or reduce vision loss; they only attempt to slow the progression of a disease already in the advanced stages. These therapies are often limited by their route of administration (1) topical - largely ineffective in posterior eye disease (2) intraocular injections - expensive, risky, limited efficacy, may directly produce blindness as a complication of the procedure, may lead to intraocular infections, requires a specialist to administer (3) IV / photodynamic therapy - requires specific equipment and administration by a specialist, limited efficacy, expensive.

Accordingly, it would be desirable to delay the onset or reduce the early development of ocular oxidative stress and resulting inflammation, thereby halting the natural progression of the disease at an early state.

### SUMMARY OF THE INVENTION

In one aspect, the invention relates to methods of using NNR ligands to delay the onset or reduce development or progression of retinopathy. In some embodiments, the NNR ligand is a modulator (e.g., agonist, partial agonist or an antagonist) of α4β2 NNRs. In some embodiments, the NNR ligand is a modulator of α7 NNRs.

In another aspect, the invention relates to the use of combinations of α4β2 ligands and α7 ligands to delay the onset or reduce the development or progression of retinopathy.

According to the methods of the present invention, in particular embodiments the retinopathy can be diabetic retinopathy or age-related macular degeneration. The methods can include screening patients to determine predisposition toward development of disorders as discussed herein and determining appropriate regimes for administration of compounds and combinations of compounds as described herein.

As used herein, the following Compound designations are as indicated: Compound A = exo-S-mecamylamine; Compound B is N-((2S,3R)-2-((pyridin-3-yl)methyl)-1-azabicyclo[2.2.2]oct-3-yl)-5-methylthiophene-2-carboxamide, illustrated below,

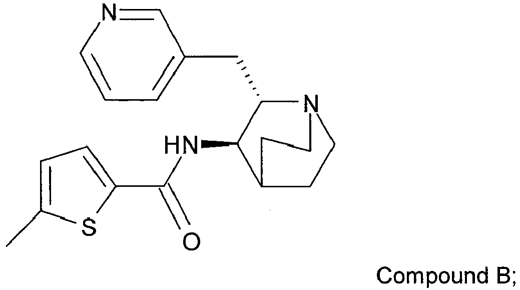

Compound C is N-((2S,3R)-2-((3-pyridinyl)methyl)-1-azabicyclo[2.2.2]oct-3-yl)-3,5-difluorobenzamide, illustrated below,

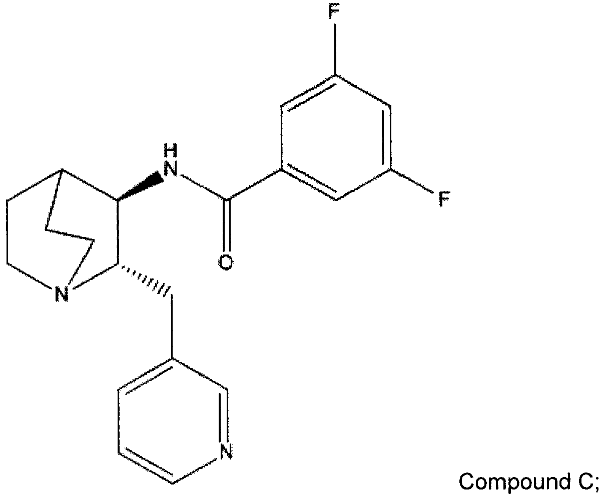

Compound D is 2-(3-pyridinyl-1-azabicyclo[2,2,2]octane, illustrated below,

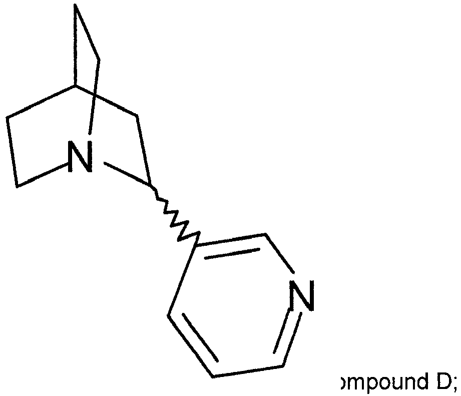

Compound E is (R)-5-((E)-2-pyrrolidin-3-ylvinyl)pyrimidine, illustrated below, 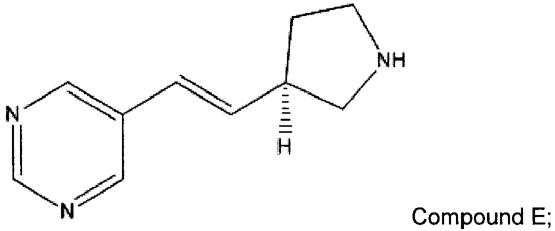 and Compounds F-H are as described below.

### BRIEF DESCRIPTION OF THE DRAWINGS

Figure 1 is a graphic representation illustrating inhibition of glutamate-induced neuronal excitotoxicity. LDH = lactate dehydrogenase; GLU = glutamate; Compounds F-H = various α4β2 ligands. See Example 1.
Figure 2 is a graphic representation illustrating that α7 NNR ligands inhibit the production of reactive oxygen species (ROS) in neuroglial cells following irradiation. DCFH = dichlorofluorescin; Gy = gray (100 rads); and a7 L = N-((2S,3R)-(2-((3-pyridinyl)methyl)-1-azabicyclo[2.2.2]oct-3-yl)-5-methylthiophene-2-carboxamide, and α7 NNR ligand.
Figure 3 is a graphic representation illustrating that transepithelial resistance deteriorates in the presence of oxidative stress (H₂O₂) and apical source of complement. Data shown is generated from ARPE-19 cell line monolayers. "HI serum" = heat inactivated serum.
Figure 4 is a graphic representation illustrating that application of nicotinic agonists or nicotine reduces transepithelial resistance (TER) deterioration in the presence of stress in a dose-dependent manner.

### DESCRIPTION OF THE INVENTION

Although preventative therapy has been implemented in cardiovascular medicine for decades (treatment of hypertension and/or high cholesterol to prevent cardiovascular morbidity), effective modalities to delay the onset of or reduce sudden vision loss in "at risk subjects" (those with prevalent soft drusen or poorly controlled diabetes) are not available. Availability of an oral medicine to delay the onset of or reduce the development of retinopathies would be a significant scientific advance because (1) no effective agent to treat or to delay the onset of or reduce retinopathies is currently available (2) oral therapies would be readily available & cheaper (i.e. increased access) as opposed to currently available therapies requiring special administrations and/or equipment, (3) would likely be more effective than alternate modes of delivery because the medication would reach the posterior retina via the choriocapillaris.

Most attempts to "treat" retinopathy have focused on managing the effects of vascular injury and growth. Some of these agents have included vascular endothelial growth factor inhibitors (VEGFi) administered topically or via direct intraocular injection (Macugen®). Complex photodynamic vascular occlusion therapy, as well as surgical laser therapy, have also been employed. All of these therapies seek to slow the progression of vision loss and do not restore vision. Once significant vascular injury / overgrowth occurs, intervention is essentially too late. Vascular effects associated with retinopathies are the result of the underlying pathology, not the cause of the disease. To create an effective therapeutic intervention for retinopathies, one must intervene early and treat the underlying cause - oxidative stress and inflammation. NNR ligands appear uniquely positioned with the proper pharmacology to elicit such effects. Furthermore, these small (MW < 500 d), basic, lipophilic amines will be readily bioavailable to the posterior retina and will achieve therapeutic concentrations within the choriocapillaris, retinal pigment epithelium and neuroretina.

The retina is replete with nicotinic receptors: Putative α7 NNRs are present in choroidal tissue; α7, α4β2*, and α6* NNRs are found in retinal ganglion cells; and α3* and α5* NNRs are found in acetylcholine-secreting amacrine cells. Early data indicate that nicotinic ligands may prevent neuronal oxidative stress, inflammation and apoptosis. Without wishing to be bound by any particular theory, it appears that oxidative stress is a precipitating factor in the development of AMD and DR. Mecamylamine, a nicotinic antagonist, appears to block NMDA receptors and prevent neuronal excitotoxicity. Therefore, α4β2 antagonists (such as those reported in US Patents 7,101,916 and 6,956,042) should also block NMDA receptors, prevent neuronal excitotoxicity, and prevent diabetic retinopathy.

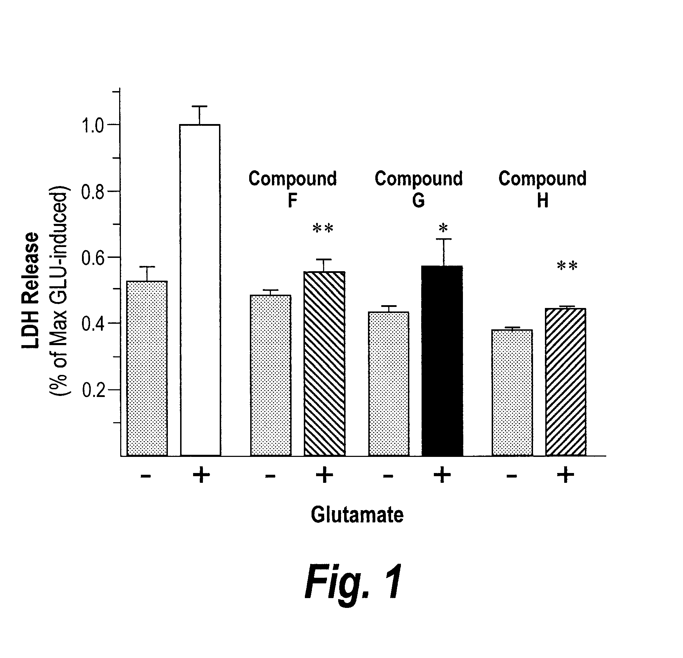

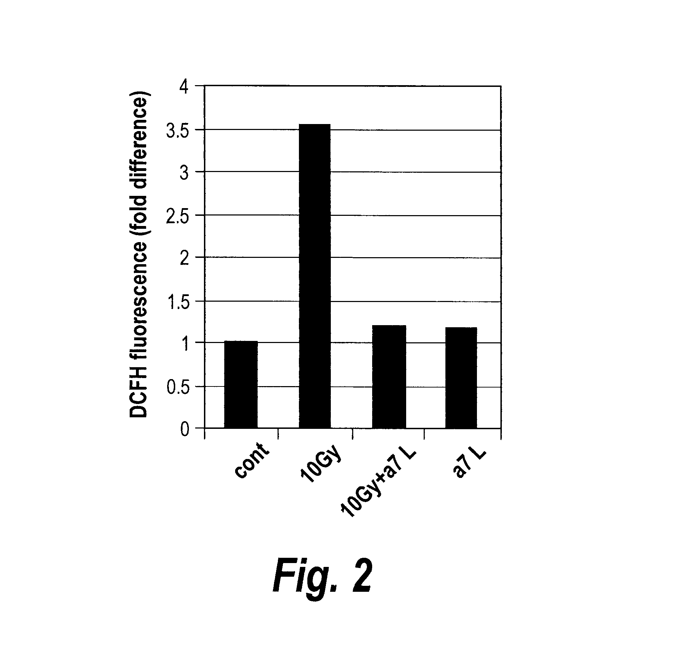

Ligands of α4β2 also appear to prevent glutamate-induced neuronal excitotoxicity (Figure 1). Furthermore, nicotinic ligands at the α7 nicotinic receptor appear to possess antiinflammatory properties, protecting neurons against injury from reactive oxygen species (Figure 2). Therefore, α7 ligands (such as those reported in US Patent 6,953,855) may protect neurons against injury from reactive oxygen species and thereby prevent macular degeneration.

Thus, one aspect of the present invention is the use of α7 ligands for the prevention of macular degeneration in persons susceptible to the disease. Also, both antagonism of NMDA receptors and stimulation of α7 nicotinic receptors appear to protect against the development of retinopathies. A combination of compounds possessing these two pharmacologies, or a single compound possessing both of these pharmacologies, could prove of therapeutic benefit in the prevention of retinopathies. Thus, another aspect of the present invention is the use of a combination of an α4β2 ligand (i.e., an antagonist) and an α7 ligand (i.e., an agonist) to delay the onset of or reduce retinopathies, especially diabetic retinopathy.

In one aspect, the invention includes methods of delaying the onset or reducing the severity of symptoms or damage caused by retinopathies, including macular degeneration and diabetic retinopathy. In particular embodiments, the methods of the invention included identifying patients susceptible to or at increased risk for development of such disorders. The beneficial effects of the method can include delaying onset or progression of the disorder, providing protective effects, and/or amelioration of symptoms.

In some embodiments of invention, such patients can be identified by the presence soft drusen in the Bruch's membrane of the eye. Macular drusen are yellow or white deposits of extracellular material and can be either hard or soft. Soft drusen can be characterized by a pale yellow color, ill-defined borders, and variability in size and shape. Soft drusen is considered to be an early indicator for propensity of a patient to develop macular degeneration and typically proceeds other characteristics associated with actual development of macular degeneration.

In particular embodiments, a patient selected on the basis of the presence of soft drusen is administered an α7 ligand. In other embodiments, such patients are administered a combination of an α4β2 antagonist and an α7 ligand. In other embodiments, such patients are administered mecamylamine, a stereoisomer thereof, or an analog thereof. The stereoisomer of mecamylamine can be exo-S-mecamylamine.

In particular embodiments, the compounds administered can be administered orally.

### α4β2 Partial Agonists

U.S. Patent Nos. 6,603,011, 5,616,716 and 6,958,399 teach compounds that are α4β2 NNR partial agonists and is incorporated herein by reference for its teaching of such compounds and methods of obtaining them.

Regarding Figure 1:
Compound F is represented by the following structure: 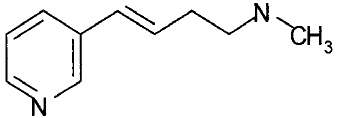
Compound G is represented by the following structure: 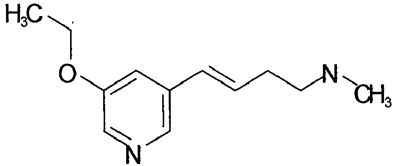
Compound H is represented by the following structure:

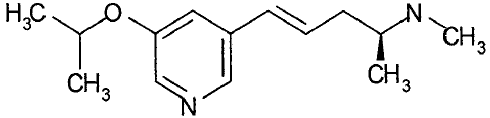

### Mecamylamine

Unless otherwise stated herein, the term "mecamylamine" means mecamylamine, its stereoisomers together as the racemic mixture and as purified separate enantiomers, analogs, the free base, and/or salts thereof. Mecamylamine can be obtained according to the methods and processes described in U.S. Patent No. 5,986,142, incorporated herein by reference for its teaching regarding method of producing mecamylamine. Purified exo-S-mecamylamine and exo-R-mecamylamine can be obtained according to methods discussed in U.S. Patent No. 7,101,916, and references cited therein, also incorporated herein by reference for their teaching regarding the production of purified mecamylamine enantiomers. Exo-S-mecamylamine is also referenced herein as Compound A.

### α7 NNR Ligands

Compounds that act as ligands of α7 NNR may be those as disclosed in U.S. Pat. No. 6,953,855, fully incorporated herein by reference for its teaching of α7 ligands and methods of obtaining the same. Such compounds are the preferred compounds of the invention.

Alpha7 NNR ligands can be identified as follows: Rats (female, Sprague-Dawley), weighing 150-250 g, were maintained on a 12 h light/dark cycle and were allowed free access to water and food supplied by PMI Nutrition International, Inc. Animals were anesthetized with 70% CO₂, then decapitated. Brains were removed and placed on an ice-cold platform. The hippocampus was removed and placed in 10 volumes (weightvolume) of ice-cold preparative buffer (137 mM NaCl, 10.7 mM KCl, 5.8 mM KH₂PO₄, 8 mM Na₂HPO₄, 20 mM HEPES (free acid), 5 mM iodoacetamide, 1.6 mM EDTA, pH 7.4); PMSF, dissolved in methanol to a final concentration of 100 µM, was added and the tissue suspension was homogenized by Polytron. The homogenate was centrifuged at 18,000 x g for 20 min at 4°C and the resulting pellet was re-suspended in 10 volumes of ice-cold water. After 60 min incubation on ice, a new pellet was collected by centrifugation at 18,000 x g for 20 min at 4°C. The final pellet was re-suspended in 10 volumes of buffer and stored at -20°C. On the day of the assay, tissue was thawed, centrifuged at 18,000 x g for 20 min, and then re-suspended in ice-cold PBS (Dulbecco's Phosphate Buffered Saline, 138 mM NaCl, 2.67 mM KCI, 1.47 mM KH₂PO₄, 8.1 mM Na₂HPO₄, 0.9 mM CaCl₂, 0.5 mM MgCl₂, Invitrogen/Gibco, pH 7.4) to a final concentration of approximately 2 mg protein/mL. Protein was determined by the method of Lowry et al., J. Biol. Chem. 193: 265 (1951), using bovine serum albumin as the standard.

The binding of [³H]MLA was measured using a modification of the methods of Davies et al., Neuropharmacol. 38: 679 (1999). [³H]MLA (Specific Activity = 25-35 Ci/mmol) was obtained from Tocris. The binding of [³H]MLA was determined using a 2 h incubation at 21°C. Incubations were conducted in 48-well micro-titre plates and contained about 200 µg of protein per well in a final incubation volume of 300 µL. The incubation buffer was PBS and the final concentration of [³H]MLA was 5 nM. The binding reaction was terminated by filtration of the protein containing bound ligand onto glass fiber filters (GF/B, Brandel) using a Brandel Tissue Harvester at room temperature. Filters were soaked in de-ionized water containing 0.33 % polyethyleneimine to reduce non-specific binding. Each filter was washed with PBS (3 x 1 mL) at room temperature. Non-specific binding was determined by inclusion of 50 µM non-radioactive MLA in selected wells.

The inhibition of [³H]MLA binding by test compounds was determined by including seven different concentrations of the test compound in selected wells. Each concentration was replicated in triplicate. IC₅₀ values were estimated as the concentration of compound that inhibited 50 percent of specific [³H]MLA binding. Inhibition constants (Ki values), reported in nM, were calculated from the IC₅₀ values using the method of Cheng et al., Biochem. Pharmacol. 22: 3099-3108 (1973).

For initial screening, a single concentration of test compounds was tested in the above assay format with the following modifications. Incubations were conducted in 96-well plates in a final incubation volume of 150 µL. Once the binding reaction was terminated by filtration onto glass fiber filters, the filters were washed four times with approximately 250 µL of PBS at room temperature. Non-specific binding was determined by inclusion of 10 µM non-radioactive MLA in selected wells. The single concentration of test compound was 5 µM and testing was performed in triplicate. 'Active' compounds were defined as compounds that inhibited the binding of [³H]MLA to the receptor by at least 50% compared with the binding of [³H]MLA in the absence of competitor. For those compounds found to be active in the single point screen, the inhibition constants (Ki values) were determined as described in the previous paragraphs of this section.

Selective α7 agonists can be found using a functional assay on FLIPR (see, for example, PCT WO 00/73431 A2, the contents of which are hereby incorporated by reference), which is a commercially available high throughput assay (Molecular Devices Corporation, Sunnyvale, California). FLIPR is designed to read the fluorescent signal from each well of a 96 or 384 well plate as fast as twice a second for up to 30 minutes. This assay can be used to accurately measure the functional pharmacology of α7 NNR and 5HT₃ subtypes. Cell lines that express functional forms of the α7 NNR subtype using the α7 /5-HT₃ channel as the drug target and/or cell lines that express functional 5-HT₃ are used to conduct the assay. In both cases, the ligand-gated ion channels are expressed in SH-EP1 cells. Both ion channels can produce a robust signal in the FLIPR assay. Using the FLIPR assay, the compounds described herein can be evaluated for their ability to function as agonists, partial agonists or antagonists at the α7 NNR subtype.

Accordingly, in one aspect, the present invention relates to a method of delaying the onset or progression of retinopathy by selecting a patient having an increased risk of developing retinopathy and administering to the patient at least one NNR ligand. The NNR ligand can be a partial agonist or an antagonist of α4β2 NNR. The NNR ligand can be an α7 NNR ligand. In some embodiments, a combination of at least one α4β2 partial agonist or antagonist and at least one α7 ligand can be administered. In particular embodiments, the at least one NNR ligand is selected from the group consisting of Compound A, Compound B, Compound C, Compound D, and Compound E. In some embodiments, the NNR ligand is Compound A. In particular embodiments, the at least one NNR ligand is administered orally.

In some embodiments, the retinopathy is diabetic retinopathy or macular degeneration.

In some embodiments, the retinopathy is age-related macular degeneration.

In another aspect, the invention relates to a method of delaying the onset or progression of retinopathy by selecting a patient by determining the presence of soft drusen in the patient's eye and administering to the patient at least one NNR ligand. In some embodiments, the NNR ligand can be a partial agonist or an antagonist of α4β2 NNR. In some embodiments, the NNR ligand is an α7 NNR ligand. In some embodiments, the at least one NNR ligand is a combination of at least one α4β2 partial agonist or antagonist and at least one α7 ligand. In particular embodiments, the at least one NNR ligand is selected from the group consisting of Compound A, Compound B, Compound C, Compound D, and Compound E. In some embodiments, the NNR ligand is Compound A.

In another aspect, the invention relates to a method for delaying or preventing hyperglycemic damage to the blood vessels of the retina comprising administering to the patient at least one NNR ligand. In some embodiments, the NNR ligand can be a partial agonist or an antagonist of α4β2 NNR. In some embodiments, the NNR ligand is an α7 NNR ligand. In some embodiments, the at least one NNR ligand is a combination of at least one α4β2 partial agonist or antagonist and at least one α7 ligand. In particular embodiments, the at least one NNR ligand is selected from the group consisting of Compound A, Compound B, Compound C, Compound D, and Compound E. In some embodiments, the NNR ligand is Compound A.

In another aspect, the invention relates to a method for treating ocular oxidative stress comprising administering to the patient at least one NNR ligand. In some embodiments, the NNR ligand can be a partial agonist or an antagonist of α4β2 NNR. In some embodiments, the NNR ligand is an α7 NNR ligand. In some embodiments, the at least one NNR ligand is a combination of at least one α4β2 partial agonist or antagonist and at least one α7 ligand. In particular embodiments, the at least one NNR ligand is selected from the group consisting of Compound A, Compound B, Compound C, Compound D, and Compound E. In some embodiments, the NNR ligand is Compound A.

In another aspect, the invention relates to a method for treating ocular inflammation comprising administering to the patient at least one NNR ligand. In some embodiments, the NNR ligand can be a partial agonist or an antagonist of α4β2 NNR. In some embodiments, the NNR ligand is an α7 NNR ligand. In some embodiments, the at least one NNR ligand is a combination of at least one α4β2 partial agonist or antagonist and at least one α7 ligand. In particular embodiments, the at least one NNR ligand is selected from the group consisting of Compound A, Compound B, Compound C, Compound D, and Compound E. In some embodiments, the NNR ligand is Compound A.

In another aspect, the invention relates to a method for providing protective effects against retinopathy comprising administering to the patient at least one NNR ligand. In some embodiments, the NNR ligand can be a partial agonist or an antagonist of α4β2 NNR. In some embodiments, the NNR ligand is an α7 NNR ligand. In some embodiments, the at least one NNR ligand is a combination of at least one α4β2 partial agonist or antagonist and at least one α7 ligand. In particular embodiments, the at least one NNR ligand is selected from the group consisting of Compound A, Compound B, Compound C, Compound D, and Compound E. In some embodiments, the NNR ligand is Compound A.

In another aspect, the invention relates to a method for modulating transepithelial resistance comprising administering to the patient at least one NNR ligand. In some embodiments, the NNR ligand can be a partial agonist or an antagonist of α4β2 NNR. In some embodiments, the NNR ligand is an α7 NNR ligand. In some embodiments, the at least one NNR ligand is a combination of at least one α4β2 partial agonist or antagonist and at least one α7 ligand. In particular embodiments, the at least one NNR ligand is selected from the group consisting of Compound A, Compound B, Compound C, Compound D, and Compound E. In some embodiments, the NNR ligand is Compound A.

The scope of the present invention includes all combinations of aspects and embodiments.

### EXAMPLES

### Testing for Protective Effect Against Light Damage-Mediated Stress

The compounds and combinations of the invention can be evaluated for their protective effect against retinal and macular dystrophies induced by light damage. Models for such testing are described in Wilson, R.B., et al., Invest. Ophthalmol. Vis. Sci., 48:2877-2886 (2007) and in Kunchithapautham, K. and B. Rohrer, Autophagy 3(5):433-441 (2007).

### Example 1 - Neuroprotection in Fetal Rat Brain - Lactate Dehydrogenase Activities

Neuroprotection studies were performed according to methods previously described (Akaike et al., Brain Res. 644:181-187 (1994). Briefly, 2- to 3-week-old cultures of fetal rat brain were exposed to test compound (10 mM) for 2 h prior to a 15-min incubation with 1 mM glutamate. Cells were assayed 24 h later for lactate dehydrogenase activities. See results in Figure 1. The results indicate that Compounds F - H are able to protect against the effects of glutamate.

### Example 2 -Alpha 7 Ligand Modulation of Radiation-Induced ROS Generation

The ability of N-((2S,3R)-(2-((3-pyridinyl)methyl)-1-azabicyclo[2.2.2]oct-3-yl)-5-methylthiophene-2-carboxamide, an α7 NNR ligand, to modulate radiation-induced generation of reactive oxygen species (ROS) was investigated. Rat brain endothelial cell line gp8.3 cells were incubated with 10 uM of the ligand for 1 h prior to irradiation with a single dose of 10 Gy. ROS generation was measured using 2'7' dichlorodihydo-fluorescein diacetate (H2DCFDA, Molecular Probes Inc., Eugene, OR) as described previously (Zhao W and Robbins, Cancer Research 61(14): 5537-43 (2001)). As shown in Figure 2, incubating gp8.3 cells with the ligand blocked radiation-induced increase in ROS level.

### Example 3 - The protective effects of nicotinic receptor activation on oxidative stress in retinal pigment epithelial (RPE) cells in vitro.

Pig primary RPE cells grown as stable monolayers were used for this set of experiments. RPE monolayers were treated with hydrogen peroxide (H2O2) and serum as a source of complement. This treatment is known to result in loss of transepithelial resistance (TER) in RPE cells that is dependent upon reactive oxygen species and superoxide production.

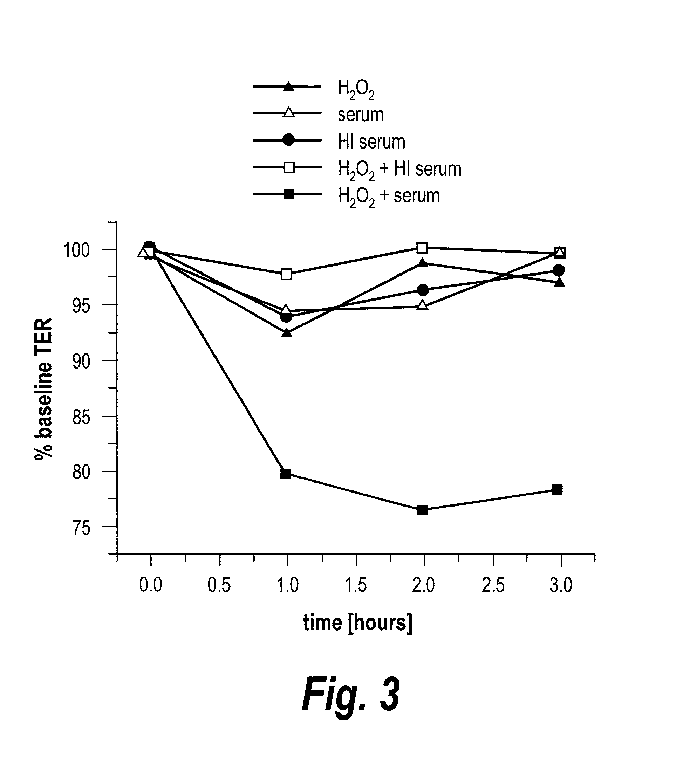

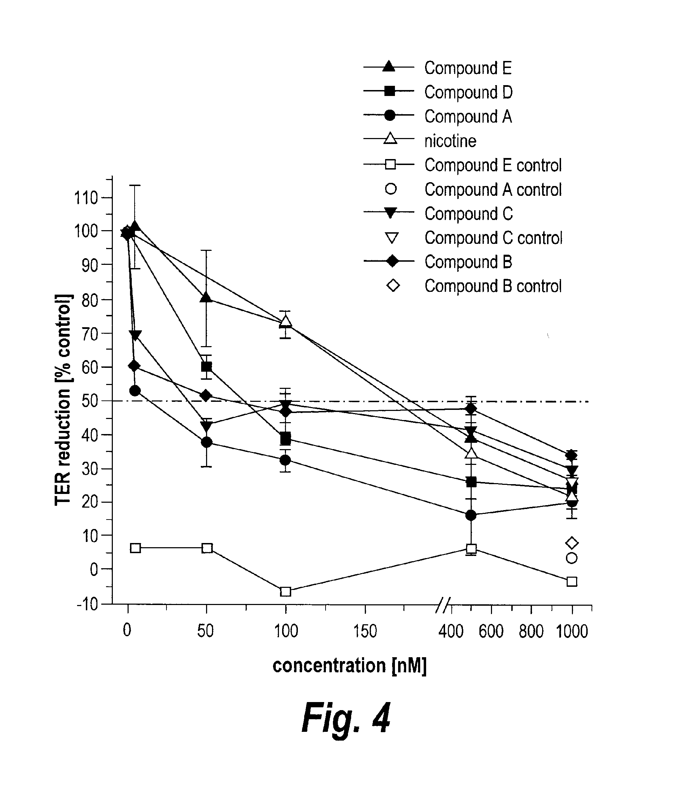

Primary pig RPE cells were grown as stable monolayers with a stable transepithelial resistance (TER) of 80-100 Ω/cm². Combined treatment of two stressors present in AMD (low oxidative stress and complement activation) results in TER loss; whereas each stressor alone has no effect. This loss in TER can be eliminated by pretreatment with superoxide dismutase (SOD) mimetic (MnTBAP). Nicotinic ligands were compared to nicotine for their ability to preserve TER upon pretreatment of monolayers for 1 hour prior to exposure to stressors. See Figure 4. Figure 3 shows data from the reference experiment evaluation of the effect of oxidative stress on RPE cell integrity. Figure 4 is derived from the maximal effects observed in Figure 3. That is, the averages for the difference between the "H₂O₂ + HI serum" and the "H₂O₂ + serum" for data points at 1, 2, and 3 hours is taken as a "100%" control relative to the data shown in Figure 4 for the test compounds and nicotine. The higher the resistance, the less injury to the cell. Note that "HI" is "heat inactivated." Heat inactivates complement, a necessary component to cause injury to the RPE in the presence of peroxides.

### Materials and Methods

Pig primary RPE cells were harvested from pigs used for practice surgeries. Cultures were performed described by Ablonczy, Z. and C.E. Crosson, VEGF modulation of retinal pigment epithelium resistance. Exp Eye Res, 2007. 85(6):762-71. Chemicals. Compounds were tested at 5 nM-1 µM concentration. Compound A -E are as noted herein.

### Results

Five nicotinic compounds, with a focus on α7 ligands, were tested for their potential to modulate TER under oxidative stress conditions. The dose response curves for the five compounds provided show that these compounds can reduce oxidative stress-mediated loss of epithelial cell integrity in a dose-dependent manner as shown in Figure 4. One compound (Compound E) acted like a nicotine-mimetic (ED50 ∼200 nM). Compound D had intermediate efficacy (ED50 ∼75 nM). The remaining three compounds had an ED50 <50 nM. Interestingly, only Compound A exhibited a dose-response profile over the entire range used; whereas the other two (Compounds B and C) plateaued at or around the ED50 level. These two compounds also reduced TER on their own, possibly suggesting independent actions of these two compounds on membrane integrity or other cellular pathways. In Figure 4, test compound controls lack H₂O₂. This control data was obtained for some test compounds only at the highest concentration level, as indicated in Figure 4.

A Fisher Posthoc test on the dose response profiles revealed that Compound E was different from the other four compounds; but due to the small sample size per treatment paradigm, no further differences could be established. See Table 1.

**Table 1: Fisher's PLSD for nicotine effect:treatment - Significance Level: 5%**

| COMPOUND | Mean Diff. | Crit. Diff. | P-Value |
|---|---|---|---|
| Compound E; Compound A | 34.222 | 17.067 | .0002 |
| Compound E; Compound C | 19.717 | 17.620 | .0293 |
| Compound E; Compound D | 24.861 | 17.067 | .0054 |
| Compound E; Compound B | 17.682 | 17.620 | .0492 |
| Compound A; Compound C | -14.503 | 18.050 | .1122 |
| Compound A; Compound D | -9.359 | 17.511 | .2863 |
| Compound A; Compound B | -16.538 | 18.050 | .0714 |
| Compound C; Compound D | 5.144 | 18.050 | .5676 |
| Compound C; Compound B | -2.035 | 18.573 | .8257 |
| Compound D; Compound B | -7.179 | 18.050 | .4260 |

When the Fisher Posthoc test was repeated on individual doses, in addition to group effect, a significant difference was revealed between Compound A and Compound C, and between Compound A and Compound B; as well as Compound A and Compound E (P<0.02). See Table 2.

**Table 2: Fisher's PLSD for 100 nM effect:treatment - Significance Level: 5%**

| COMPOUND | Mean Diff. | Crit. Diff. | P-Value |
|---|---|---|---|
| Compound E; Compound A | 39.927 | 10.435 | .0002 |
| Compound E; Compound C | 23.652 | 10.435 | .0021 |
| Compound E; Compound D | 33.750 | 10.435 | .0004 |
| Compound E; Compound B | 25.718 | 10.435 | .0014 |
| Compound A; Compound C | -16.276 | 10.435 | .0102 |
| Compound A; Compound D | -6.177 | 10.435 | .1886 |
| Compound A; Compound B | -14.209 | 10.435 | .0173 |
| Compound C; Compound D | 10.098 | 10.435 | .0553 |
| Compound C; Compound B | 2.066 | 10.435 | .6324 |
| Compound D; Compound B | -8.023 | 10.435 | .1048 |

### Examples 4 and 5 - Oxidative Stress in dry AMD and Laser Damage

Two animal models for AMD are used that focus on different aspects of the disease; constant light-damage in albino mice for oxidative stress in dry AMD (see Rohrer B, et al., Lack of p75 receptor does not protect photoreceptors from light-induced cell death. Exp Eye Res 2003, 76(1):125-129) and laser damage (choroidal neovascularization - CNV) in pigmented mice for angiogenesis in wet AMD (see Nozaki M, et al., Drusen complement components C3a and C5a promote choroidal neovascularization. Proc Natl Acad Sci. U S A 2006, 103(7):2328-2333). Compounds are examined using subcutaneous injections. The effects of the nicotinic ligands are assessed using electroretinography to assess photoreceptor function and histology to assess tissue integrity. In particular, chronic light-exposed animals are assessed for cell counts in photoreceptor and RPE layers; CNV-treated animals for the size of the CNV lesion (Isolectin B staining of the vasculature). Additional data can include ELISA assays for oxidative stress and VEGF quantification, and quantitative PCR to quantify changes in mRNA for angiogenic and cell death determinants.

Mice. C57BU6 and Balb/c mice raised under normal 12:12 cyclic light condition (25-30 lux) are used.

Light damage. Based on the fact that the lack of complement activation only has an effect on the susceptibility to light-damage in old but not juvenile mice (Fig. 3), the effect of treatment is assessed in aged (15-monthold) mice. Animals are moved into the light-treatment room where they are housed separately in clear Plexiglas cages with free access to food and water. Light-damage is induced by exposing the animals to 150-175 lux of white light provided by two 30 Watt fluorescent bulbs (General Electric; T30T12-CW-RS) suspended ∼40 cm above the cages (see Richards A, et al., Long-term ERG analysis in the partially light-damaged mouse retina reveals regressive and compensatory changes, Vis Neurosci 2006, 23(1):91-97). This amount of light reduces the number of rods to 5 rows within 10 days in albino mice (see Lohr HR, et al., Multiple, parallel cellular suicide mechanisms participate in photoreceptor cell death. Exp Eye Res 2006, 83(2):380-389). Light damage can require 6-10 animals per experimental condition to obtain statistically significant data for basic electroretinography and histology.

CNV. 3-month-old animals (C57BL/6) will be anesthetized using xylazine and ketamine (20 and 80 mg/kg, respectively) and pupils dilated with a drop of phenylephrine HCl (2.5%) and atropine sulfate (1%). Argon laser photocoagulation (532 nm, 50 µm spot size, 0.05 s duration, 250 mW) will be used to generate four laser spots in each eye surrounding the optic nerve, using a handheld coverslip as a contact lens. A bubble formed at a laser spot will indicate the rupture of Bruch's membrane (Nozaki, M., *et al*. (2006)). CNV can requires 8-10 animals per experimental condition to obtain statistically significant data for basic electroretinography and histology.

Subcutaneous injections. Injections are made using a 25-G needle and injecting a volume of 0.2 mL.

Treatment schedules. Experimental solutions contain the compound of choice diluted in PBS: control solutions contain PBS only. Doses are varied in ½ log unit steps around the starting dose (at least 3 doses, or more if required) to determine the optimum dose.

Electroretinography. ERG recordings are performed in dark-adapted animals to assess rod and cone function. Function is correlated with photoreceptor numbers and RPE integrity obtained by histology (Richards A, *et al.,* (2006)). ERG amplitudes, in particular b-wave amplitudes, are extremely sensitive to oxygen supply to the retina, such that indirect recordings can be used to assess RPE/choroid integrity. This can be used in lieu of RPE c-wave recordings, which are extremely complex in the mouse eye (Wu J, et al., Functional abnormalities in the retinal pigment epithelium of CFTR mutant mice. Exp Eye Res 2006, 83(2):424-428). Histology. Retinal and RPE/choroid integrity are assessed in histological sections of epon-embedded tissue at the light microscopic level (Rohrer B, Korenbrot JI, LaVail MM, Reichardt LF, Xu B: Role of neurotrophin receptor TrkB in the maturation of rod photoreceptors and establishment of synaptic transmission to the inner retina. J Neurosci. 1999, 19(20):8919-8930). This preparation is used to perform cell counts in photoreceptor and RPE layer.

Assessment of CNV lesions. CNV size is determined in flat-mount preparations of RPE/choroids stained with isolectin B (which binds_to terminal β-D-galactose residues on the surface of endothelial cells and selectively labels the murine_vasculature) (Nozaki, M., *et al*. (2006)). Fluorescence measurements taken in 2 µm sections using confocal microscopy are used_for volume determination.

Follow-up studies on optimal dose:

Quantitative RT-PCR. QRT-PCR is used to quantify changes in mRNA for complement components and angiogenic determinants (see Lohr, HR, *et al.,* (2006)). In particular, message for VEGF and its receptor VEGF-R1 and R2, TGF-_2 are assessed in the RPE-choroid fraction for CNV; caspase 3, BIRC3, clusterin and ceruloplasmin are analyzed in the retina fraction for light damage. Four control and four experimental animals are used for these assays.

ELISA assays. To confirm that the angiogenic and complement components are indeed upregulated at the protein level, ELISAs are performed for VEGF and TGF-_2 quantification. ROS and O₂⁻ are measured using the dichlorofluorescein diacetate and dihydroethidium dye (Molecular Probes). Cells (5 x 105) are loaded with the dye (10 µM) for 15 min and fluorescence is monitored by fluorometery at an excitation/emission wavelength of 480/520 nm. Four control and four experimental animals are used for these assays.

Data interpretation. Data assembled is initially evaluated with univariate statistics to assure that the quality of the data is adequate for further analyses. Each measured parameter (e.g., CNV size, % cell loss, etc.) is correlated against dose of compound provided. The dependent variable in these analyses is % reversal of light or laser damage.

Certain aspects of the invention are summarized in the following numbered paragraphs:
1. A method of delaying the onset or progression of retinopathy, the method comprising:
   a) selecting a patient having an increased risk of developing retinopathy;
   b) administering to the patient at least one NNR ligand.
2. The method of paragraph 1, wherein the NNR ligand is a partial agonist or an antagonist of α4β2 NNR.
3. The method of paragraph 1, wherein the NNR ligand is an α7 NNR ligand.
4. The method of paragraph 1, wherein the at least one NNR ligand is a combination of at least one α4β2 partial agonist or antagonist and at least one α7 ligand.
5. The method of paragraph 1, wherein the at least one NNR ligand is selected from the group consisting of Compound A, Compound B, Compound C, Compound D, and Compound E.
6. The method of paragraph 1, wherein the at least one NNR ligand is Compound A.
7. The method of paragraph 1, wherein the retinopathy is selected from the group consisting of diabetic retinopathy and macular degeneration.
8. The method of paragraph 1, wherein the retinopathy is age-related macular degeneration.
9. The method of paragraph 1, wherein the at least one NNR ligand is administered orally.
10. A method of delaying the onset or progression of retinopathy, the method comprising:
   a) selecting a patient by determining the presence of soft drusen in the Bruch's membrane of the patient's eye;
   b) administering to the patient at least one NNR ligand.
11. The method of paragraph 10, wherein the NNR ligand is a partial agonist or an antagonist of α4β2 NNR.
12. The method of paragraph 10, wherein the NNR ligand is an α7 NNR ligand.
13. The method of paragraph 10, wherein the at least one NNR ligand is a combination of at least one α4β2 partial agonist or antagonist and at least one α7 ligand.
14. The method of paragraph 10, wherein the at least one NNR ligand is selected from the group consisting of Compound A, Compound B, Compound C, Compound D, and Compound E.
15. The method of paragraph 10, wherein the at least one NNR ligand is Compound A.
16. A method for treating ocular oxidative stress comprising administering to the patient at least one NNR ligand.
17. A method for treating ocular inflammation comprising administering to the patient at least one NNR ligand.
18. A method for providing protective effects against retinopathy comprising administering to the patient at least one NNR ligand.
19. A method for modulating transepithelial resistance comprising administering to the patient at least one NNR ligand.
20. A method for delaying or preventing hyperglycemic damage to the blood vessels of the retina comprising administering to the patient at least one NNR ligand.
21. The method of any one of paragraphs 16-20, wherein the NNR ligand is a partial agonist or an antagonist of α4β2 NNR.
22. The method of any one of paragraphs 16-20, wherein the NNR ligand is an α7 NNR ligand.
23. The method of any one of paragraphs 16-20, wherein the at least one NNR ligand is a combination of at least one α4β2 partial agonist or antagonist and at least one α7 ligand.
24. The method of any one of paragraphs 16-20, wherein the at least one NNR ligand is selected from the group consisting of Compound A, Compound B, Compound C, Compound D, and Compound E.
25. The method of any one of paragraphs 16-20, wherein the at least one NNR ligand is Compound A.

## Claims

1. The use of exo-S-mecamylamine in the manufacture of a medicament for oral administration to delay the onset or progression of retinopathy in a patient having an increased risk of developing retinopathy.

2. The use of claim 1, wherein the medicament is for delaying the onset or progression of retinopathy in a patient with soft drusen in the Bruch's membrane.

3. The use of exo-S-mecamylamine in the manufacture of a medicament for oral administration for the treatment of ocular oxidative stress, ocular inflammation, for providing protective effects against retinopathy, modulating transepithelial resistance, or for delaying or preventing hyperglycemic damage to the blood vessels of the retina.

4. The use of any one of claims 1 to 3, wherein exo-S-mecamylamine is a partial agonist or an antagonist of α4β2 NNR and/or an α7 NNR ligand.

5. The use of claim 1 or 2, wherein the retinopathy is selected from the group consisting of diabetic retinopathy and macular degeneration and age-related macular degeneration.

6. Exo-S-mecamylamine for use in oral administration to delay the onset or progression of retinopathy in a patient having an increased risk of developing retinopathy.

7. Exo-S-mecamylamine according to claim 6 for use to delay the onset or progression of retinopathy in a patient with soft drusen in the Bruch's membrane.

8. Exo-S-mecamylamine for use in oral administration for the treatment of ocular oxidative stress, ocular inflammation, for providing protective effects against retinopathy, modulating transepithelial resistance, or for delaying or preventing hyperglycemic damage to the blood vessels of the retina.

9. Exo-S-mecamylamine according to any one of claims 6 to 8, which is a partial agonist or an antagonist of α4β2 NNR and/or an α7 NNR ligand.

10. Exo-S-mecamylamine according to claims 6 or 7, wherein the retinopathy is selected from diabetic retinopathy and macular degeneration and age-related macular degeneration.
